(19) Europäisches Patentamt
European Patent Office
Office européen des brevets

(11) **EP 4 563 117 A1**

(12) **EUROPÄISCHE PATENTANMELDUNG**

(43) Veröffentlichungstag:
    **04.06.2025  Patentblatt 2025/23**

(21) Anmeldenummer: **23213096.3**

(22) Anmeldetag: **29.11.2023**

(51) Internationale Patentklassifikation (IPC):
    *A61C 13/00* (2006.01)    *A61C 13/08* (2006.01)
    *A61C 13/083* (2006.01)    *A61K 6/00* (2020.01)
    *A61K 6/818* (2020.01)    *B33Y 10/00* (2015.01)
    *B33Y 40/20* (2020.01)    *B33Y 80/00* (2015.01)

(52) Gemeinsame Patentklassifikation (CPC):
    **A61C 13/0019; A61C 13/082; A61C 13/083;
    B33Y 10/00; B33Y 40/20; B33Y 80/00;** A61K 6/802;
    B33Y 70/00

(84) Benannte Vertragsstaaten:
    **AL AT BE BG CH CY CZ DE DK EE ES FI FR GB
    GR HR HU IE IS IT LI LT LU LV MC ME MK MT NL
    NO PL PT RO RS SE SI SK SM TR**
    Benannte Erstreckungsstaaten:
    **BA**
    Benannte Validierungsstaaten:
    **KH MA MD TN**

(71) Anmelder: **Ivoclar Vivadent AG
    9494 Schaan (LI)**

(72) Erfinder:
    • **John, Hendrik
      9470 Buchs (CH)**
    • **Rothbrust, Frank
      6822 Röns (AT)**
    • **Krolikowski, Sebastian
      8805 Richterswil (CH)**
    • **Ritzberger, Christian
      9472 Grabs (CH)**

(74) Vertreter: **Baldus, Oliver
    Splanemann
    Rumfordstrasse 7
    80469 München (DE)**

(54) **SELEKTIVE EINFÄRBUNG MITTELS FÄRBELÖSUNGEN BEIM 3D-DRUCK**

(57)    Die vorliegende Erfindung betrifft ein Verfahren zum Herstellen einer dentalen Restauration (100) durch Strahldruck, mit den Schritten eines Strahldruckens (S101) einer oder mehrerer Schichten der dentalen Restauration mittels eines keramischen Schlickers; und ei- nes Strahldruckens (S102) einer Färbelösung auf die eine oder mehreren Schichten.

Fig. 7

**Beschreibung**

Die vorliegende Erfindung betrifft ein Verfahren zum Herstellen einer dentalen Restauration durch Strahldruck und ein Herstellungsgerät zum Herstellen einer dentalen Restauration durch Strahldruck.

Verschieden eingefärbte, lösungsmittelbasierte Keramikschlicker, wie beispielsweise aus Zirkondioxid, können selektiv mittels eines Strahlverfahrens (Inkjet-Verfahren) tröpfchenweise in einer Vielzahl von Schichten aufeinander aufgetragen werden. Die Verarbeitung von hochgefülltem, keramischem Schlicker stellt jedoch hohe Anforderungen an den eingesetzten Druckkopf in Bezug auf eine Partikelgröße, einen Füllgrad, eine Viskosität und Abrasion. Das Strahldrucken (Jetten) von wässrigen Keramikschlickern stellt eine Alternative zu den lösungsmittelbasierten Schlickern dar. Dabei wird die gejettete Materialschicht rissfrei getrocknet.

Es ist die technische Aufgabe der vorliegenden Erfindung, ein Multi-Color-3D-Druckverfahren mittels Inkjet für keramische Schlicker zu verbessern.

Diese Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch ein Verfahren zum Herstellen einer dentalen Restauration durch Strahldruck gelöst, mit den Schritten eines Strahldruckens einer oder mehrerer Schichten der dentalen Restauration mittels eines keramischen Schlickers; und eines Strahldruckens einer Färbelösung auf die eine oder mehreren Schichten. Dadurch kann der selektive Auftragsprozess des Schlickers und ein selektiver Einfärbeprozess voneinander getrennt werden. Für den Auftragsprozess und den Einfärbeprozess können die gleichen oder unterschiedliche Druckköpfe eingesetzt werden.

Werden demgegenüber bereits voreingefärbte Schlicker verwendet, die zudem auch noch unterschiedliche Transluzenzen und/oder unterschiedliche mechanische Eigenschaften haben, so wird jedem dieser Schlicker ein eigener Druckkopf zugeordnet. Durch das Verfahren kann demgegenüber die Anzahl an Druckköpfen zum Auftragen der Schlicker verringert werden, da die Farbgebung getrennt von dem Schlickeraufbau durch das nachträgliche Strahldrucken von Färbelösungen erfolgt.

In einer technisch vorteilhaften Ausführungsform des Verfahrens werden die eine oder mehreren Schichten vor dem Strahldrucken der Färbelösung getrocknet. Dadurch wird beispielsweise der technische Vorteil erreicht, dass die Färbelösung weniger diffundiert und sich die örtliche Homogenität der Einfärbung verbessert.

In einer weiteren technisch vorteilhaften Ausführungsform des Verfahrens wird die aufgebrachte Färbelösung mittels einer Lauge fixiert. Die Lauge kann nach oder vor dem Auftrag der Färbelösung auf die Schicht aufgetragen werden. Dadurch wird beispielsweise der technische Vorteil erreicht, dass ein Verlaufen der Färbelösung verhindert wird.

In einer weiteren technisch vorteilhaften Ausführungsform des Verfahrens weist der keramische Schlicker einen basischen pH-Wert auf. Dadurch wird beispielsweise der technische Vorteil erreicht, dass die Färbelösung automatisch durch den Schlicker fixiert wird.

In einer weiteren technisch vorteilhaften Ausführungsform des Verfahrens wird die Färbelösung auf die eine oder mehreren feuchten Schichten strahlgedruckt. Dadurch wird beispielsweise der technische Vorteil erreicht, dass es zu einer Fällungsreaktion der Färbelösung kommt und das Verfahren schneller durchgeführt werden kann.

In einer weiteren technisch vorteilhaften Ausführungsform des Verfahrens wird die Färbelösung durch Kontakt mit dem keramischen Schlicker fixiert. Dadurch wird beispielsweise der technische Vorteil erreicht, dass das Verfahren effizienter durchgeführt wird.

In einer weiteren technisch vorteilhaften Ausführungsform des Verfahrens werden die eine oder mehreren feuchten Schichten zusammen mit der aufgebrachten Färbelösung getrocknet. Dadurch wird beispielsweise der technische Vorteil erreicht, dass unmittelbar die nächste Schicht aufgebracht werden kann.

In einer weiteren technisch vorteilhaften Ausführungsform des Verfahrens werden die Schritte zum Herstellen der dentalen Restauration wiederholt. Dadurch wird beispielsweise der technische Vorteil erreicht, dass die gesamte dentale Restauration aufgebaut und eingefärbt werden kann.

In einer weiteren technisch vorteilhaften Ausführungsform des Verfahrens wird die dentale Restauration in einem Sinterofen gesintert. Dadurch wird beispielsweise der technische Vorteil erreicht, dass eine dentale Restauration mit hoher Festigkeit hergestellt werden kann.

In einer weiteren technisch vorteilhaften Ausführungsform des Verfahrens wird die hergestellte dentale Restauration vor einem Sinterprozess einem Trocknungs- und/oder Entbinderungsschritt unterzogen. Dieser Trocknungs- und/oder Entbinderungsschritt kann in einem separaten thermischen Prozess erfolgen oder ist ein im Sinterprozess vorgelagerter Prozessschritt. Üblicherweise wird das Trocknen bei Temperaturen von 25°C bis 200°C, bevorzugt 30°C bis 180°C und besonders bevorzugt 40°C bis 150°C getrocknet. Dabei kann zusätzlich die Luftfeuchtigkeit zwischen 10 bis 90%, bevorzugt 15 bis 85% und besonders bevorzugt 20 bis 80% reguliert werden. Üblicherweise wird bei Temperaturen von 50°C bis 600°C, bevorzugt zwischen 100°C bis 600°C und besonders bevorzugt bei 200°C-600°C entbindert. Die Aufheizgeschwindigkeiten liegen zwischen 0.1 bis 10K/min, bevorzugt zwischen 0.2 bis 10K/min und besonders bevorzugt zwischen 0.5 bis 10K/min.

Gemäß einem zweiten Aspekt wird die technische Aufgabe durch ein Herstellungsgerät zum Herstellen einer dentalen Restauration durch Strahldruck gelöst, mit einem ersten Druckkopf zum Strahldrucken einer oder mehrerer Schichten der dentalen Restauration mittels eines keramischen Schlickers; und einem zweiten Druckkopf zum Strahldrucken einer Färbelösung auf die eine oder mehreren Schichten. Durch das Herstellungsgerät werden die gleichen technischen Vorteile wie durch das Verfahren nach dem ersten Aspekt erreicht.

In einer technisch vorteilhaften Ausführungsform des Herstellungsgeräts sind der erste Druckkopf und der zweite Druckkopf in einem gemeinsamen Druckmodul integriert. Dadurch wird beispielsweise der technische Vorteil erreicht, dass sich der Aufbau des Herstellungsgerätes vereinfacht.

In einer weiteren technisch vorteilhaften Ausführungsform des Herstellungsgeräts sind der erste Druckkopf und der zweite Druckkopf unabhängig voneinander steuerbar. Dadurch wird beispielsweise der technische Vorteil erreicht, dass das Auftragen des Schlickers und der Färbelösung unabhängig voneinander erfolgen können.

In einer weiteren technisch vorteilhaften Ausführungsform des Herstellungsgeräts umfasst das Herstellungsgerät ein Dithering-Modul zum Berechnen von Farbzwischenwerten durch Mischen von mindestens zwei Färbelösungen. Dadurch wird beispielsweise der technische Vorteil erreicht, dass sich das naturgetreue Aussehen der dentalen Restauration weiter verbessert.

In einer weiteren technisch vorteilhaften Ausführungsform des Herstellungsgeräts ist das Dithering-Modul ausgebildet, in aufeinanderfolgenden Schichten der dentalen Restauration unterschiedliche zweidimensionale Dithering-Muster zu verwenden. Dadurch wird beispielsweise der technische Vorteil erreicht, dass das Entstehen von Streifen- oder Moiré-Mustern in der dentalen Restauration verhindert wird.

Ausführungsbeispiele der Erfindung sind in den Zeichnungen dargestellt und werden im Folgenden näher beschrieben.

Es zeigen:

Fig. 1    eine Darstellung unterschiedlicher Bereiche eines Schneidezahns;

Fig. 2    eine schematische Darstellung einer dentalen Restauration;

Fig. 3    eine schematische Darstellung eines Herstellungsgeräts zum Herstellen einer dentalen Restauration;

Fig. 4    eine schematische Ansicht eines Verfahrens zum Herstellen einer dentalen Restauration durch Strahldruck;

Fig. 5    eine schematische Ansicht eines weiteren Verfahrens zum Herstellen einer dentalen Restauration durch Strahldruck;

Fig. 6    eine Tabelle mit den Zusammensetzungen unterschiedlicher Färbelösungen; und

Fig. 7    ein Blockdiagramm des Verfahrens zum Herstellen einer dentalen Restauration durch Strahldruck.

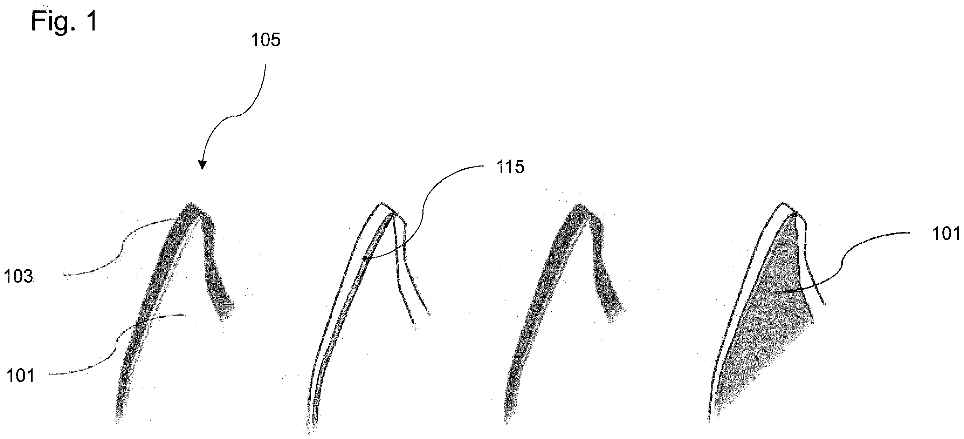

Fig. 1 zeigt eine Darstellung unterschiedlicher Bereiche eines Zahns 105. Der Zahn 105 umfasst einen inneren Dentinkern 101 und einen äußeren Zahnschmelz 103. Zwischen dem Dentinkern 101 und dem Zahnschmelz 103 befindet sich optional ein Zwischenbereich 115, dem ebenfalls eine eigene Auswahl an keramischen Schlickern 109 und Färbelösungen 107 zugewiesen werden kann.

Für die Grundeinfärbung des Zahns 105 ist der opake, d.h. undurchsichtige Dentinkern 101 verantwortlich. Dieser scheint durch den Zahnschmelz 103 der Schneide hindurch. Der Zahnschmelz 103 ist naturgemäß durchscheinend, d.h. transluzent. Transluzenz ist die partielle Lichtdurchlässigkeit eines Körpers. Um eine dentale Restauration möglichst naturgetreu aussehen zu lassen, wird dieser Aufbau des Zahns 105 auch in künstlichen dentalen Restaurationen verwendet. Hierzu werden Materialien mit unterschiedlichen optischen Eigenschaften bei der Herstellung der dentalen Restauration verwendet.

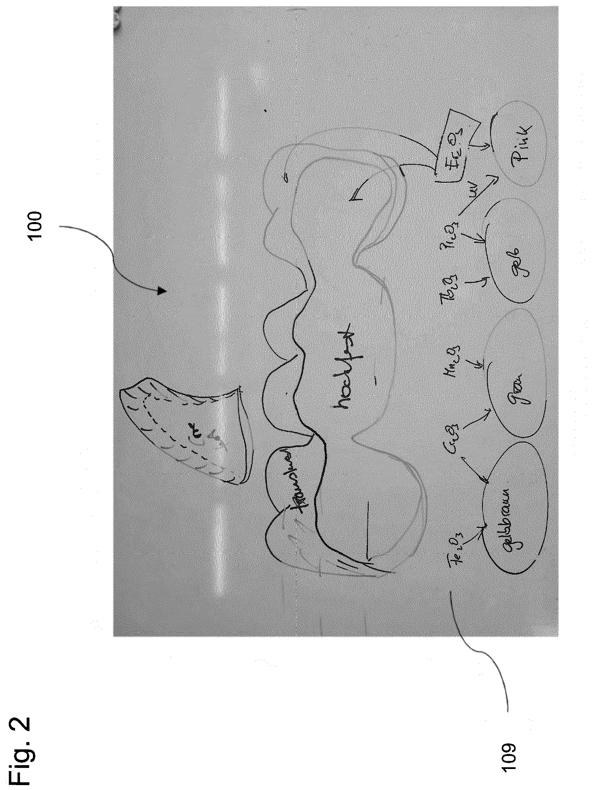

Fig. 2 zeigt eine schematische Darstellung einer dentalen Restauration 100. Die dentale Restauration 100 dient als Zahnersatz und ist beispielsweise durch eine Brücke, Krone, Veneer, Inlay, Onlay, Abutment, Voll- oder Teilprothese gebildet. Die dentale Restauration 100 wird beispielsweise mittels unterschiedlicher keramischer Schlicker 109 aufgebaut. Der keramische Schlicker umfasst teilstabilisierten $ZrO_2$ und/oder $Al_2O_3$-Partikel. $Al_2O_3$ kann analog der Stabi-lisierungsionen bereits eingebaut sein. Die Teilstabilisierung erfolgt üblicherweise durch Einbau von $CaO$, $MgO$, $Y_2O_3$, $La_2O_3$ oder $CeO_2$ sowie deren Mischungen im $ZrO_2$. Eine bevorzugte Ausführungsform enthält $Y_2O_3$ als Stabilisator im Mengenbereich von 1-10 Mol.%, besonders bevorzugtem Mengenbereich von 2-8 Mol.% und ganz besonders bevorzugtem Mengenbereich von 2.5-6 Mol.%. Hierzu werden diese Schlicker 109 bei der räumlichen Herstellung der dentalen Restauration 100 mittels eines Strahldruckverfahrens selektiv in den jeweiligen Bereichen eingesetzt.

Die dentale Restauration 100 wird dabei durch aufeinanderfolgende Schichten aufgebaut, die aufeinander gedruckt werden. Dabei können die Keramikpulver der Schlicker 109 bereits in vorgegebenen Transluzenzen bereitgestellt sein. Durch die Mischung dieser Schlicker 109 ergibt sich die Soll-Transluzenz der dentalen Restauration 100 in dem jeweiligen räumlichen Bereich.

Nach dem selektiven Materialauftrag einer Schicht des Schlickers 109 mittels des Strahldruckverfahrens wird diese Schicht rissfrei getrocknet, indem das Wasser oder das Lösungsmittel als Binder verdampft wird. Zurück bleibt eine poröse Weissling-Schicht, die eine Schichtdicke von 1 um bis 50 um und eine Dichte von mindestens 2.5 g/cm3 aufweist. Dieser Prozess wird wiederholt, bis die gesamte dentale Restauration 100 schichtweise räumlich aufgebaut ist.

Im Falle von ZrO2-Schlickern 109 können für die verschiedenen Festigkeiten unterschiedliche Yttrium-dotierte Keramikpulver (3 mol% Yttrium - 3Y-TZP, 4 mol% Yttrium - 4Y-TZP, 5 mol% Yttrium - 5Y-TZP) zum Einsatz kommen.

```
3Y-TZP = niedrige Transluzenz / hohe Festigkeit

4Y-TZP = mittlere Transluzenz / mittlere Festigkeit

5Y-TZP = hohe Transluzenz / niedrige Festigkeit
```

Werden jedoch unterschiedlich voreingefärbte Schlicker 109 verwendet, die alle auch eine unterschiedliche Transluzenz und/oder mechanische Eigenschaften aufweisen, ist jedem dieser Schlicker 109 ein eigener Druckkopf 111-1 zugeordnet. Wird beispielsweise ein allgemeines Vierfarben-Farbschema, wie beispielsweise CMKY (Cyan, Magenta, Yellow, Key/White), eingesetzt, um den gesamten Farbraum abzudecken und zudem Schlicker mit unterschiedlichen Transluzenzen oder Festigkeiten in drei Varianten eingesetzt, so sind 3 x 4 = 12 Schlicker 109 in 12 Druckköpfen enthalten. Auch die Herstellung dieser verschiedenen Schlicker 109 ist aufwendig. Diese große Anzahl an Schlickern 109 wird als unterschiedliche Artikel vorgehalten und gepflegt werden.

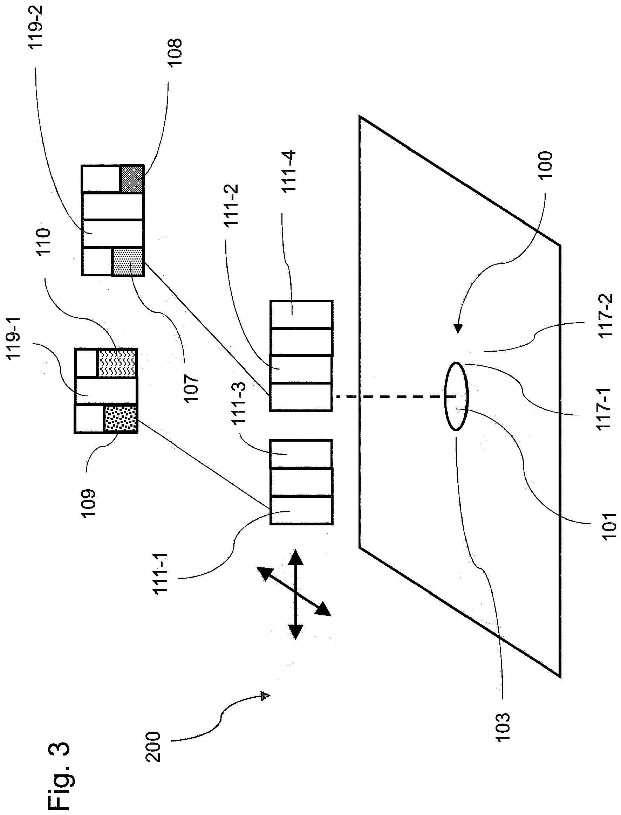

Fig. 3 zeigt eine schematische Darstellung eines Herstellungsgeräts 200 zum Herstellen einer dentalen Restauration 100 durch Strahldruck (Inkjet-Druck) von wässrigen oder lösungsmittelbasierten Schlickern 109. Um keramische, dentale Multimaterial- und Multicolor-Restaurationen 100 mittels Strahldruck additiv zu fertigen, werden Basis-Schlicker 109 verarbeitet.

Das Herstellungsgerät 200 umfasst eine Mehrzahl von Aufnahmebehältern 119-1, in denen die Basis-Schlicker 109 mit unterschiedlichen optischen Eigenschaften und das Stützmaterial 110 angeordnet sind. Zudem umfasst das Herstellungsgerät 200 zumindest mehrere Aufnahmebehälter 119-2, in denen die Färbelösungen 107 und die Lauge 108 zur Fixierung der Färbelösung aufgenommen sind.

Die keramischen Schlicker 109 werden jeweils mittels eines zugeordneten Druckkopfes 111-1 tröpfchenweise in mehreren Schichten 117-1, ..., 117-n aufgebracht, um die dentale Restauration 100 schichtweise räumlich aufzubauen. Der Druckkopf 111-1 ist in zwei Richtungen beweglich, so dass der Schlicker 109 an jeder Position aufgedruckt werden kann. Für den selektiven Materialauftrag werden Schlicker 109 mit einem Tropfenvolumen von typischerweise 10 bis 100 pL verwendet, durch deren Einsatz der zeitlich aufwändige Entbinderungsprozess entfällt. Zum Ausstoßen der Tropfen werden beispielsweise elektrisch gesteuerte Piezoelemente verwendet. Auch der Einsatz von Bubble-Jet Technologie ist zum Ausstoßen der Schlickertropfen möglich. Für die Auftragung des Stützmaterials 110 wird mindestens ein weiterer Druckkopf 111-3 verwendet, durch den das Stützmaterial 110 selektiv aufgetragen werden kann.

Für die Auftragung der Färbelösungen 107 wird mindestens ein weiterer Druckkopf 111-2 verwendet, durch den die Färbelösungen 107 auf eine oder mehrere Schichten 117-1, ..., 117-n strahlgedruckt werden können. Der Druckkopf 111-2 ist ebenfalls in zwei Richtungen beweglich, so dass die Färbelösungen 107 an jeder Position aufgedruckt werden können.

Optional wird für die Auftragung der Lauge 108 zum Fixieren der Färbelösungen 107 mindestens ein weiterer Druckkopf 111-4 verwendet, durch den die Lauge 108 auf eine oder mehrere Schichten 117-1, ..., 117-n strahlgedruckt werden können. Der Druckkopf 111-2 ist ebenfalls in zwei Richtungen beweglich, so dass die Lauge 108 an jeder Position aufgedruckt werden können. Die Druckköpfe 111-1, 111-2, 111-3 und 111-4 können unabhängig voneinander steuerbar sein oder in einem gemeinsamen Druckmodul integriert sein.

Das Herstellungsgerät 200 stellt eine verringerte Anzahl an ggf. voreingefärbten und Yttrium-dotierten neutralen Basis-Schlickern 109 bereit, um die Anzahl der Druckköpfe 111-1 und 111-2 zu minimieren und dennoch ein ästhetisches und funktionelles Ergebnis der dentalen Restauration 100 zu erzielen. Die finale Farbgebung erfolgt getrennt durch das selektive Aufbringen von Färbelösungen 107.

Die gewünschte Zahnfarbe wird aus den unterschiedlichen Färbelösungen 107 zusammengesetzt und gemischt. Dabei wird ein subtraktives Farbsystem verwendet, das einen eingeschränkten, dentalen Farbraum (Dental Color Gamut) aufspannt. Die Farbmischung, das dreidimensionale Halftoning oder Dithering dieser Färbelösungen 107 in verschiedenen Verhältnissen, entsteht dann in dem eingeschränkten, dentalen Farbraum (Gamut), der die gängigen Zahnfarben, jedoch nicht alle Farben abdeckt.

Dabei werden unterschiedliche Färbelösungen 107 über einen 3D-Dithering-Algorithmus auf eine Schicht 117-1, ..., 117-n selektiv aufgetragen, der durch ein Dithering-Modul 113 ausgeführt wird. Das Dithering-Modul 113 umfasst hierzu einen Prozessor zum Ausführen des 3D-Dithering-Algorithmus und einen digitalen Datenspeicher zum Speichern der berechneten Mischungsverhältnisse und des Dithering-Algorithmus. Der Prozessor umfasst jedes Hardwaresystem, jede Komponente oder jeden Mechanismus, der Daten, Signale oder andere Informationen verarbeitet. Ein Prozessor kann ein System mit einer zentralen Datenverarbeitungseinheit (CPU), mehreren Datenverarbeitungseinheiten (MPU), eine dedizierte elektrische Schaltung zum Erreichen der Funktionalität oder andere Systeme umfassen. Der Datenspeicher kann Festplatten, Flash-Speicherkarten, wahlfreie Zugriffsspeicher (RAM) oder Nur-Lesespeicher (ROM) umfassen.

Bei dem Dithering werden die unterschiedlichen Färbelösungen in einem bestimmten Verhältnis und zweidimensionalen Druckmuster in der Druckebene selektiv mittels des Strahldruckverfahrens aufgetragen. Der 3D-Dithering-Algorithmus berechnet zudem, dass nicht gleiche zweidimensionale Dithering-Muster in mehreren Schichten 117-1, ..., 117-n übereinander aufgetragen werden. Dadurch können bei senkrechten Flächen optische Artefakte wie Streifen- oder Moiré-Muster verhindert werden. Die Farbmischung, das dreidimensionale Halftoning oder Dithering dieser Färbelösungen 107 in verschiedenen Verhältnissen entsteht innerhalb des eingeschränkten, dentalen Farbraums (Gamut), der die gängigen Zahnfarben, jedoch nicht alle allgemeinen Farben abdeckt.

Bei dem Einsatz von hochviskosen Schlickern 109 mit einer Viskosität von mehr als 100 mPas mit einem hohen Füllgrad werden besondere Anforderungen an den Druckkopf 111-1 und sein Fluidsystem gestellt. Der Druckkopf 111-1 ist mit dem verwendeten Binder und/oder dem Trägermaterial des Schlickers 109 kompatibel, wie beispielsweise Wasser bei wässrigen Schlickern, um Unverträglichkeiten und Folgen wie beispielsweise Korrosion am Druckkopf zu vermeiden.

Nach dem selektiven Materialauftrag einer Schicht 117-1, ..., 117-n des Schlickers 109 mittels des Strahldruckverfahrens wird diese Schicht 117-1, ..., 117-n rissfrei getrocknet, indem das Wasser oder das Lösungsmittel als Binder verdampft wird. Dabei kann das Keramikpulver des Schlickers 109 bereits in einer Farbe eingefärbt sein, beispielsweise einer Grundfarbe oder Zahnfarbe. Zurück bleibt eine poröse Weissling-Schicht, die eine Schichtdicke von 1 um bis 50 um und eine Dichte von mindestens 2.5 g/cm$^3$ aufweist. Dieser Prozess wird wiederholt, bis die gesamte dentale Restauration 100 schichtweise räumlich aufgebaut ist.

Stehen zwei Schlicker 109 mit unterschiedlichen Transluzenzen zur Verfügung, kann der hochfeste und opake Schlicker 109 für den Dentinkern und der normalfeste und hochtransluzente Schlicker 109 für die Schneide eingesetzt werden. Die selektive Einfärbung erfolgt jeweils nach 1-n Schichten 117-1, ..., 117-n des Materialauftrags durch das selektive Strahldrucken von Färbelösungen, wie beispielsweise Nitrat-Lösungen (Säuren), nach einer entsprechenden Farbkodierung oder Farbinformation.

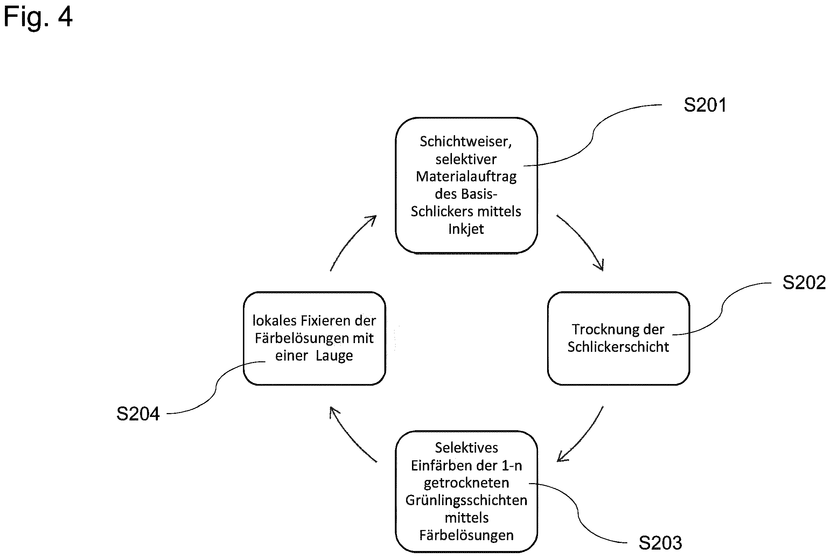

Fig. 4 zeigt eine schematische Ansicht eines Verfahrens zum Herstellen der dentalen Restauration 100 durch Strahldruck. Im Schritt S201 erfolgt zunächst ein schichtweiser selektiver Materialauftrag eines Schlickers 109 mittels eines Strahldruckverfahrens (Inkjet-Verfahren). In Schritt S102 wird die aufgebrachte Schlickerschicht getrocknet, beispielsweise durch Zuführen von Warmluft. In Schritt S203 werden die 1 bis n aufgebrachten Schichten 117-1, ..., 117-n des Schlickers 109 (Grünlingsschichten) mittels Färbelösungen selektiv eingefärbt. Hierbei werden die Färbelösungen ebenfalls mittels Strahldruck auf die 1 bis n aufgebrachten Schichten 117-1, ..., 117-n aufgedruckt. In Schritt S204 werden die aufgedruckten Färbelösungen mittels einer Lauge 108 lokal fixiert, d.h. mittels einer alkalischen Lösung mit einem pH-Wert, der größer als 7 ist. Anschließend werden die Schritte S201 bis S204 so lange wiederholt, bis die dentale Restauration 100 vollständig räumlich aufgebaut ist.

Für das Verfahren können entweder wässrige oder lösungsmittelbasierte Schlicker 109 verarbeitet werden. Ein selektiver Materialauftrag (Schritt S201) und eine selektive Einfärbung (Schritt S203) erfolgen dabei getrennt.

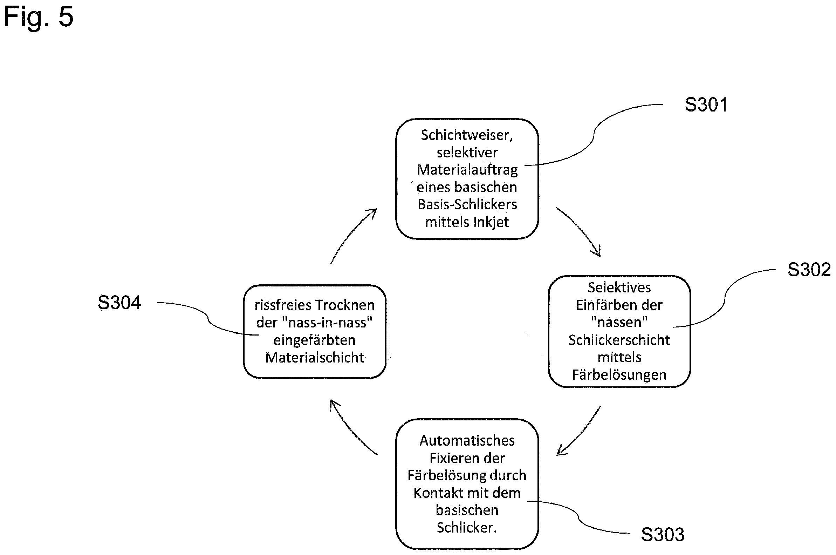

Fig. 5 zeigt eine schematische Ansicht eines weiteren Verfahrens zum Herstellen der dentalen Restauration 100 durch Strahldruck. Im Schritt S301 erfolgt zunächst ein schichtweiser selektiver Materialauftrag eines basischen Schlickers 109 mittels eines Strahldruckverfahrens (Inkjet-Verfahren).

Der basische Schlicker 109 weist einen pH-Wert von größer als 7 auf. Ein üblicher basischer Schlicker 109 enthält H2O, Dispergierhilfsmittel, wie beispielsweise 0 bis 5 Gew.-%, bevorzugt 0.01 bis 5 Gew.% Carbonsäurederivate (Zitronensäure) oder 0 bis 5 Gew.-% Ammoniumpolyacrylsäure (NH4PAA), bevorzugt 0.01 bis 5Gew. %. Der pH-Wert (8-11) kann mit NH4OH eingestellt werden. Die basische Einstellung des Schlickers 109 hat den Vorteil, dass sich der keramische Schlicker 109 weniger aggressiv in Bezug auf Korrosion gegenüber den Metallteilen des Druckkopfes verhält.

Des Weiteren kann der Schlicker geringe Mengen, wie beispielsweise 0 bis 5 Gew.%, bevorzugt 0.001 bis 5 Gew.%, an Verdickungsmittel zur Einstellung der Viskosität und/oder des Sedimentationsverhaltens enthalten, wie z.B. Polyvinylpyrolidon, Cellusolederivate oder Xanthan. Das Sedimentationsverhalten bewirkt, dass die Suspension über einen längeren Zeitraum stabil ist und der Sedimentation entgegenwirkt.

Im Schritt S302 werden die noch nassen und ungetrockneten aufgebrachten Schichten 117-1, ..., 117-n des Schlickers 109 (Grünlingsschichten) mittels der Färbelösungen selektiv eingefärbt. Dabei kommt es zu einer Fällungsreaktion, sobald die Färbelösung (Nitratsäure) auf den feuchten Schlicker 109 auftrifft. Gelöste Ionen der 3d-und 4f-Elemente werden durch die pH-Wert-Verschiebung beim Auftreffen auf die feuchte Schicht als Hydroxide ausgefällt. Es bilden sich hierbei z.B. Fe(OH)3 oder Er(OH)3.

Im Schritt S303 werden die Färbelösungen durch den Kontakt mit dem basischen Schlicker 109 automatisch fixiert. In Schritt S304 werden die nass in nass eingefärbten Schichten 117-1, ..., 117-n rissfrei getrocknet, beispielsweise durch Zuführen von Warmluft. Anschließend werden die Schritte S301 bis S304 so lange wiederholt, bis die dentale Restauration 100 vollständig räumlich aufgebaut ist.

Für das Verfahren können entweder wässrige oder lösungsmittelbasierte Schlicker 109 verarbeitet werden. Ein selektiver Materialauftrag (Schritt S301) und eine selektive Einfärbung (Schritt S302) erfolgen dabei getrennt.

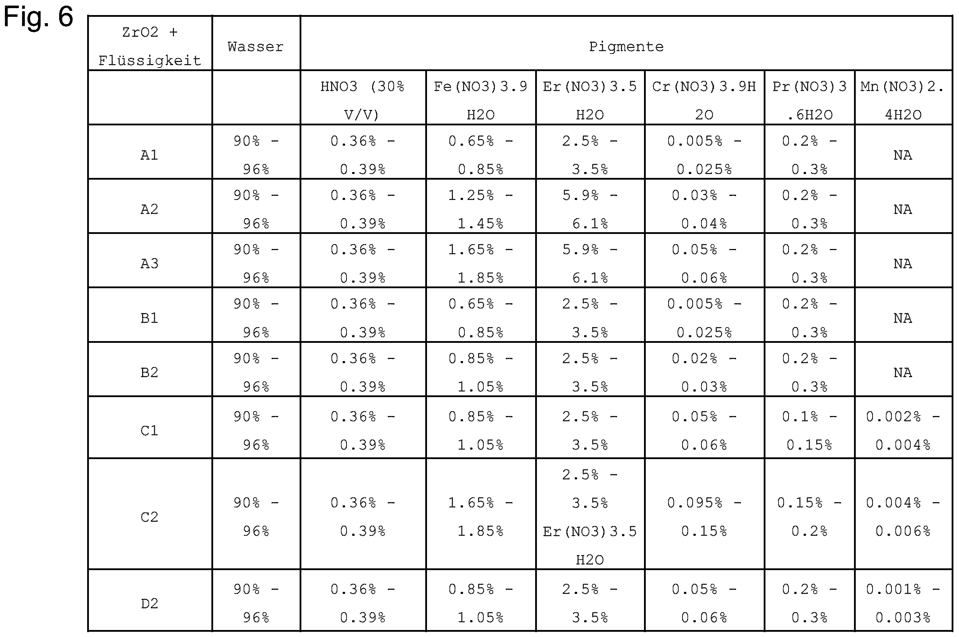

Fig. 6 zeigt eine Tabelle mit den Zusammensetzungen unterschiedlicher Färbelösungen 107. Die Färbelösungen 107 sind niederviskos und können entweder mittels eines Tintenstrahl-Druckkopfes mit DOD-Technologie oder Bubble-Jet-Technologie strahlgedruckt werden. In einem Druckkopf können für einen Multifarbdruck mehrere Farbkanäle integriert sein. Der Druckkopf kann die Färbelösung in einer hohen Auflösung von $\geq 720$ dpi auf die Schicht 117-1, ..., 117-n aufbringen.

Bei sechs verfügbaren Druckköpfen wird nur ein einziger Druckkopf für Schlicker 109 mittlerer Transluzenz und ein Druckkopf für Stützmaterial 110 verwendet. Bei sieben verfügbaren Druckköpfen werden zwei Druckköpfe für jeweils einen opaken Schlicker 109 und einen hochtransluzenten Schlicker 109 und ein Druckkopf für Stützmaterial 110 verwendet. Die jeweils anderen vier Druckköpfe werden für die Färbelösungen 107 verwendet.

Die Färbelösungen 107 sind Nitratsalz- oder Chloridsalzlösungen auf wässriger Basis. In der Färbelösung 107 sind verschiedene Metallsalze (z.B. Fe(NO3)3*9 H2O; Pr(NO3)3*6 H2O; Tb(NO3)3*5 H2O; Er(NO3)3*5 H2O; Mn(NO3) 2*4 H2O; Co(NO3)2*6 H2O; Cr(NO3)3*9 H2O; Mg(NO3)2*6 H2O; Al(NO3)3*9 H2O; Cu(NO3)2*3 H2O; Zn(NO3)2*6 H2O; Y(NO3)3*6 H2O; La(NO3)3*6 H2O; Ce(NO3)3*6 H2O; Nd(NO3)3*6 H2O; Sm(NO3)3*6 H2O; Gd(NO3)3*6 H2O; Yb(NO3)3*6 H2O); Ni(NO3)2* 6H2O; Co(NO3)2 *6H2O; Ga(NO3)3* xH2O; In(NO3)3 xH2O) in unterschiedlichen Konzentrationen gelöst. Die Konzentration hängt von der gewünschten Farbintensität ab, sollte aber die Löslichkeitsgrenze der Salze nicht überschreiten. Die Färbelösungen 107 können aber je nach gewünschter Zusammensetzung auch deutlich höher konzentriert sein. Durch eine Steigerung der Ionen-Konzentration steigt aber eine Viskosität an oder der pH-Wert verschiebt sich in den sauren Bereich (<7). Eine höhere Viskosität der Färbelösung ist von Vorteil, um die Eindringtiefe oder Diffusionstiefe der Färbelösung in die getrockneten Schichten zu reduzieren.

Als Basissäure kann Wasser und Salpetersäure (HNO3) verwendet werden. Somit werden nur vier Nitratsalzlösungen (Fe(NO3)3.9H2O, Er(NO3)3.5H2O, Cr(NO3)3.9H2O, Mn(NO3)2 *4H2O, Tb(NO3)3 *5H2O und Pr(NO3) 3.6H2O) als Färbelösung 107 verwendet, um die Zahnfarbe zu erzeugen. Minimal werden nur drei Nitratsalzlösungen (Fe-gelb, Er-pink, Cr-grau) benötigt. Die anderen Salze dienen der feineren Farbeinstellung.

Zusätzlich kann durch variable Bestandteile von Yttrium oder Ytterbium in der Färbeflüssigkeit die Opazität des Schlickers 109 der dentalen Restauration 100 eingestellt werden. So werden beispielsweise die Elemente Yttrium, Ytterbium, Neodym und Europium als Transluzenz-Verstärker (Translucency Enhancer) und Aluminium und Silizium als opakes Liquid eingesetzt. Mit einer geeigneten Färbelösung kann der Yttrium-Gehalt erhöht werden. Somit können unterschiedliche Opazitätswerte in der dentalen Restauration 100 erreicht werden, wie beispielsweise opak für den Dentinkern und transluzent für die Schneide.

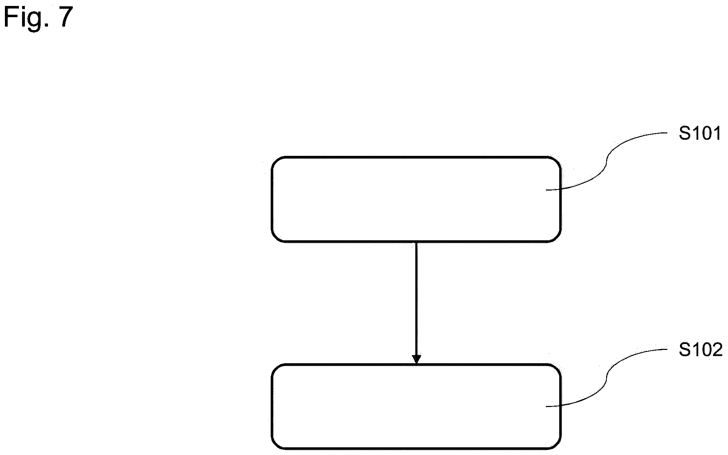

Fig. 7 zeigt ein Blockdiagramm des Verfahrens zum Herstellen einer dentalen Restauration 100 durch Strahldruck. Im Schritt S101 werden eine oder mehrere Schichten 117-1, ..., 117-n der dentalen Restauration 100 mittels des keramischen Schlickers 109 strahlgedruckt. In Schritt S102 wird anschließend die Färbelösung 107 auf die Schichten 117-1, ..., 117-n strahlgedruckt.

Ist die Infiltrationstiefe der Färbelösung 107 größer als eine Dicke einer einzelnen Schicht 117-1, ..., 117-n, kann die Einfärbung erst nach mehrfachem Schichtauftrag des Schlickers 109 erfolgen. Die Eindring- oder Diffusionstiefe der Färbelösung 107 kann außerdem über die Einstellung der Viskosität gesteuert werden. Dies erfolgt durch Zusatz eines geeigneten Verdickungsmittels, das im pH-Bereich der Färbelösung stabil ist. Ein geeignetes Verdickungsmittel ist beispielsweise Polyvinylpyrolidon (PVP). Die Eindring- oder Diffusionstiefe aller verwendeten Färbelösungen können auf ein einheitliches Niveau gebracht werden, um einen möglichst stabilen Prozess zu erzielen.

Ein Einsatz unterschiedlicher Druckkopftechnologien ist sinnvoll, da die Schlicker 109 und die Färbelösung 107 unterschiedliche rheologische Eigenschaften aufweisen. Der Schlicker 109 ist hochviskos (10 - 1000 mPas abhängig von der Scherrate) und beinhaltet einen hohen Gewichts-/Volumenanteil eines abrasiven Füllstoffs in Form von Keramikpartikeln. Vorteilhaft ist eine niedrige Viskosität des Schlickers 109 zwischen 10 - 500 mPas und noch vorteilhafter ist eine Viskosität zwischen 10 - 100 mPas. Je höher die Viskosität des Schlickers 109 ist, desto schwieriger ist die Verarbeitung mittels Inkjet-Druckköpfen basierend auf MEMS-Technologie.

Die Färbelösung 107 ist dagegen niedrigviskos (0.5 - 50 mPas). Zudem sind die jeweils verarbeiteten Volumen unterschiedlich und liegen in einem Mengenverhältnis von ca. 98% für den Schlicker 109 und bei 2% für die Färbelösung 107. Das Hauptvolumen wird über den Schlicker 109 verarbeitet, wohingegen von der Färbelösung 107 weniger Volumen verarbeitet wird. Die Färbelösung 107 und der Schlicker 109 können in unterschiedlich großen Aufnahmebehältern 119-1 und 119-2 gespeichert werden.

Das Strahldrucken der Färbelösung 107 kann nach der Trocknung der aufgetragenen Schichten 117-1, ..., 117-n erfolgen oder auf der nassen Schicht 117-1, ..., 117-n durchgeführt werden (nassin-nass). Bei dem Strahldrucken der Färbelösung 107 auf die getrocknete Schicht 117-1, ..., 117-n sollte die Eindringtiefe der Färbelösung 107 kontrollierbar sein.

Danach wird die vollständig aufgebaute dentale Restauration 100 in einem Sinterofen gesintert. Die Schlicker 109 in Verbindung mit den Färbelösungen 107 können auf ein einheitliches Sinterverhalten abgestimmt sein. Die Sinterkinetik kann durch eine Anpassung der Färbelösung 107 homogen eingestellt werden.

Die Einstellung des Sinterverhaltens der einzelnen Schichten kann durch gezielte Zugabe von Sinteraktivatoren oder Sinterinhibitor in die Färbelösung erfolgen. Sinteraktivatoren sind z.B. $Zn^{2+}$-, $Al^{3+}$- oder $Mg^{2+}$-Ionen die in Form von löslichen Salzen, wie z.B. $Zn(NO3)_2*6H_2O$, $Al(NO3)_3 * 9H_2O$ oder $Mg(NO3) *H_2O$ der Färbelösung zugegeben werden können. Sinterinhibitoren sind z.B. $La^{3+}$ oder $Y^{3+}$ die in Form von löslichen Salzen, wie z.B. $La(NO3)_3 * 9H_2O$ oder $Y(NO3)_3 * 6H_2O$ der Färbelösung zugegeben werden können.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

BEZUGSZEICHENLISTE

| | |
|---|---|
| 100 | Dentale Restauration |
| 101 | Dentinkern |
| 103 | Zahnschmelz |
| 105 | Zahn |
| 107 | Färbelösung |
| 108 | Lauge |
| 109 | Schlicker |
| 110 | Stützmaterial |
| 111 | Druckkopf |
| 113 | Dithering-Modul |
| 115 | Zwischenbereich |
| 117 | Schicht |
| 119 | Aufnahmebehälter |

**Patentansprüche**

1. Verfahren zum Herstellen einer dentalen Restauration (100) durch Strahldruck, mit den Schritten:

   - Strahldrucken (S101) einer oder mehrerer Schichten (117-1, ..., 117-n) der dentalen Restauration (100) mittels eines keramischen Schlickers (109); und
   - Strahldrucken (S102) einer Färbelösung (107) auf die eine oder mehreren Schichten (117-1, ..., 117-n).

2. Verfahren nach Anspruch 1, wobei die eine oder mehreren Schichten (117-1, ..., 117-n) vor dem Strahldrucken der Färbelösung (107) getrocknet werden.

3. Verfahren nach einem der vorangehenden Ansprüche, wobei die aufgebrachte Färbelösung (107) mittels einer Lauge (108) fixiert wird.

4. Verfahren nach einem der vorangehenden Ansprüche, wobei der keramische Schlicker (109) einen basischen pH-Wert aufweist.

5. Verfahren nach Anspruch 4, wobei die Färbelösung (107) durch Kontakt mit dem keramischen Schlicker (109) fixiert wird.

6. Verfahren nach Anspruch 4 oder 5, wobei die eine oder mehreren feuchten Schichten (117-1, ..., 117-n) zusammen mit der aufgebrachten Färbelösung (107) getrocknet werden.

7. Verfahren nach einem der vorangehenden Ansprüche, wobei die Schritte zum Herstellen der dentalen Restauration (100) wiederholt werden.

8. Verfahren nach einem der vorhergehenden Ansprüche, wobei die hergestellte dentale Restauration (100) vor einem Sinterprozess einem Trocknungs- und/oder Entbinderungsschritt unterzogen wird.

9. Verfahren nach einem der vorangehenden Ansprüche, wobei die dentale Restauration (100) in einem Sinterofen gesintert wird.

10. Herstellungsgerät (200) zum Herstellen einer dentalen Restauration (100) durch Strahldruck, mit:

   - einem ersten Druckkopf (111-1) zum Strahldrucken einer oder mehrerer Schichten (117-1, ..., 117-n) der dentalen Restauration (100) mittels eines keramischen Schlickers (109); und
   - einem zweiten Druckkopf (111-2) zum Strahldrucken einer Färbelösung (107) auf die eine oder mehreren Schichten (117-1, ..., 117-n).

11. Herstellungsgerät (200) nach Anspruch 10, wobei das Herstellungsgerät (200) einen Aufnahmebehälter (119-1) für den Schlicker (109) und einen Aufnahmebehälter (119-2) für die Färbelösung (107) umfasst.

12. Herstellungsgerät (200) nach Anspruch 10 oder 11, wobei der erste Druckkopf (111-1) und der zweite Druckkopf (111-2) in einem gemeinsamen Druckmodul (121) integriert sind.

13. Herstellungsgerät (200) nach einem der Ansprüche 10 bis 12, wobei der erste Druckkopf (111-1) und der zweite Druckkopf (111-2) unabhängig voneinander steuerbar sind.

14. Herstellungsgerät (200) nach einem der Ansprüche 10 bis 13, wobei das Herstellungsgerät (200) ein Dithering-Modul (113) zum Berechnen von Farbzwischenwerten durch Mischen von mindestens zwei Färbelösungen (107) umfasst.

15. Herstellungsgerät (200) nach Anspruch 13, wobei das Dithering-Modul (113) ausgebildet ist, in aufeinanderfolgenden Schichten (117-1, ..., 117-n) der dentalen Restauration (100) unterschiedliche zweidimensionale Dithering-Muster zu verwenden.

EP 4 563 117 A1

Fig. 1

# Fig. 2

Fig. 3

Fig. 4

S201 — Schichtweiser, selektiver Materialauftrag des Basis-Schlickers mittels Inkjet

S202 — Trocknung der Schlickerschicht

S203 — Selektives Einfärben der 1-n getrockneten Grünlingsschichten mittels Färbelösungen

S204 — lokales Fixieren der Färbelösungen mit einer Lauge

Fig. 5

S301 — Schichtweiser, selektiver Materialauftrag eines basischen Basis-Schlickers mittels Inkjet

S302 — Selektives Einfärben der "nassen" Schlickerschicht mittels Färbelösungen

S303 — Automatisches Fixieren der Färbelösung durch Kontakt mit dem basischen Schlicker.

S304 — rissfreies Trocknen der "nass-in-nass" eingefärbten Materialschicht

Fig. 6

| ZrO2 + Flüssigkeit | Wasser | Pigmente | | | | | |
|---|---|---|---|---|---|---|---|
| | | HNO3 (30% V/V) | Fe(NO3)3.9 H2O | Er(NO3)3.5 H2O | Cr(NO3)3.9H 2O | Pr(NO3)3 .6H2O | Mn(NO3)2. 4H2O |
| A1 | 90% – 96% | 0.36% – 0.39% | 0.65% – 0.85% | 2.5% – 3.5% | 0.005% – 0.025% | 0.2% – 0.3% | NA |
| A2 | 90% – 96% | 0.36% – 0.39% | 1.25% – 1.45% | 5.9% – 6.1% | 0.03% – 0.04% | 0.2% – 0.3% | NA |
| A3 | 90% – 96% | 0.36% – 0.39% | 1.65% – 1.85% | 5.9% – 6.1% | 0.05% – 0.06% | 0.2% – 0.3% | NA |
| B1 | 90% – 96% | 0.36% – 0.39% | 0.65% – 0.85% | 2.5% – 3.5% | 0.005% – 0.025% | 0.2% – 0.3% | NA |
| B2 | 90% – 96% | 0.36% – 0.39% | 0.85% – 1.05% | 2.5% – 3.5% | 0.02% – 0.03% | 0.2% – 0.3% | NA |
| C1 | 90% – 96% | 0.36% – 0.39% | 0.85% – 1.05% | 2.5% – 3.5% | 0.05% – 0.06% | 0.1% – 0.15% | 0.002% – 0.004% |
| C2 | 90% – 96% | 0.36% – 0.39% | 1.65% – 1.85% | 2.5% – 3.5% Er(NO3)3.5 H2O | 0.095% – 0.15% | 0.15% – 0.2% | 0.004% – 0.006% |
| D2 | 90% – 96% | 0.36% – 0.39% | 0.85% – 1.05% | 2.5% – 3.5% | 0.05% – 0.06% | 0.2% – 0.3% | 0.001% – 0.003% |

EP 4 563 117 A1

Fig. 7

S101

S102

EP 4 563 117 A1

Europäisches
Patentamt

European
Patent Office

Office européen
des brevets

# EUROPÄISCHER RECHERCHENBERICHT

Nummer der Anmeldung

EP 23 21 3096

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 2 529 694 A1 (IVOCLAR VIVADENT AG [LI]) 5. Dezember 2012 (2012-12-05) | 1,7-13 | INV. A61C13/00 |
| A | * Absätze [0001], [0024], [0032], [0046], [0059], [0060] * | 14,15 | A61C13/08 A61C13/083 |
| | ----- | | A61K6/00 |
| X | EP 3 659 547 A1 (IVOCLAR VIVADENT AG [LI]) 3. Juni 2020 (2020-06-03) | 1-6,10 | A61K6/818 B33Y10/00 |
| A | * Absätze [0001], [0014] – [0016], [0023], [0026], [0028], [0044], [0050], [0051], [0056], [0058] * | 14,15 | B33Y40/20 B33Y80/00 |
| | ----- | | |

RECHERCHIERTE
SACHGEBIETE (IPC)

A61C
A61K
B33Y

Der vorliegende Recherchenbericht wurde für alle Patentansprüche erstellt

| Recherchenort | Abschlußdatum der Recherche | Prüfer |
|---|---|---|
| München | 10. April 2024 | Kerner, Bodo |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder  Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes
Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 23 21 3096

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-04-2024

| Im Recherchenbericht angeführtes Patentdokument | Datum der Veröffentlichung | Mitglied(er) der Patentfamilie | Datum der Veröffentlichung |
|---|---|---|---|
| EP 2529694 A1 | 05-12-2012 | EP 2529694 A1 | 05-12-2012 |
| | | JP 2012250022 A | 20-12-2012 |
| | | US 2012308837 A1 | 06-12-2012 |
| EP 3659547 A1 | 03-06-2020 | CN 111233466 A | 05-06-2020 |
| | | EP 3659547 A1 | 03-06-2020 |
| | | ES 2906273 T3 | 18-04-2022 |
| | | JP 7128168 B2 | 30-08-2022 |
| | | JP 2020083755 A | 04-06-2020 |
| | | KR 20200066225 A | 09-06-2020 |
| | | KR 20230040967 A | 23-03-2023 |
| | | PL 3659547 T3 | 21-03-2022 |
| | | US 2020171699 A1 | 04-06-2020 |
| | | US 2023347548 A1 | 02-11-2023 |

EPO FORM P0461

Für nähere Einzelheiten zu diesem Anhang : siehe Amtsblatt des Europäischen Patentamts, Nr.12/82